(19) Europäisches Patentamt
European Patent Office
Office européen des brevets

(11) Numéro de publication: **0 341 123**
**A2**

(12) # DEMANDE DE BREVET EUROPEEN

(21) Numéro de dépôt: **89401136.0**

(22) Date de dépôt: **21.04.89**

(51) Int. Cl.[4]: **C 08 F 20/36**
C 07 C 93/04, C 07 C 149/24

(30) Priorité: **29.04.88 FR 8805801**

(43) Date de publication de la demande:
**08.11.89 Bulletin 89/45**

(84) Etats contractants désignés:
**AT BE DE FR GB IT NL SE**

(71) Demandeur: **ATOCHEM**
**4 & 8, Cours Michelet La Défense 10**
**F-92800 Puteaux (FR)**

(72) Inventeur: **Parsy, Roland**
**Résidence Hélène Boucher R19 25,Rue Hélène Boucher**
**F-76600 Le Havre (FR)**

**Augustin, Daniel**
**Corneville La Fouquetière**
**F-27300 Bernay (FR)**

**Collette, Christian**
**19, Rue Vauquelin**
**F-75005 Paris (FR)**

(74) Mandataire: **Foiret, Claude et al**
**ATOCHEM Département Propriété Industrielle**
**F-92091 Paris la Défense 10 Cédex 42 (FR)**

(54) **Nouvel oligomère dérivé d'amine grasse polyéthoxylée-son procédé de fabrication-son application pour la modification des propriétés superficielles des polymères.**

(57) Oligomère de formule :

$$\underset{O=C\,-(O-C_2H_4-NH-\underset{\underset{O}{\|}}{C})_b-(O-C_2H_4)_n-\underset{\underset{(C_2H_4-O)_{n'}-H}{|}}{N}-R_2-(Z)_m-R_3}{\overset{-(CR_1-CH_2)_a-}{|}}$$

où : $R_1$ = H ou $CH_3$, $R_2 = C_tH_{2t}$ avec $0 \leqq t \leqq 18$, Z est choisi parmi

$$C_2H_4,\ C_2H_2 \text{ et } \underset{S-C_2H_4-C_dF_{2d+1}}{\underset{|}{CH}}-CH_2$$

avec $2 \leqq d \leqq 16$, m est un nombre entier tel que $1 \leqq m \leqq 3$, $R_3$ = $C_{t'}H_{2t'+1}$ avec $t' \geqq 1$ et $8 \leqq t + t' + 2m \leqq 22$, n et n' sont des nombres entiers, identiques ou différents, tels que $2 \leqq n + n' \leqq 20$, b est un nombre entier égal à O ou 1, a est un nombre entier variant entre 2 et 500.

L'oligomère est obtenu par polymérisation d'un monomère résultant de la condensation ou de l'addition d'une amine grasse polyéthoxylée ou d'un mélange d'amines grasses polyéthoxylées sur un monomère à insaturation acrylique. L'oligomère peut être utilisé comme antibuée dans les films de polymère thermoplastique.

EP 0 341 123 A2

## Description

# NOUVEL OLIGOMERE DERIVE D'AMINE GRASSE POLYETHOXYLEE -SON PROCEDE DE FABRICATION - SON APPLICATION POUR LA MODIFICATION DES PROPRIETES SUPERFICIELLES DES POLYMERES

La présente invention concerne un nouvel oligomère dérivé d'amine grasse polyéthoxylée. Cet oligomère à propriété tensioactive est principalement utilisé pour modifier les propriétés superficielles des polymères et plus particulièrement utilisé comme antibuée. Il est obtenu par polymérisation d'un monomère issu de la condensation ou de l'addition d'une amine grasse polyéthoxylée sur un monomère à insaturation acrylique ou méthacrylique.

Il est connu de modifier les propriétés superficielles d'un polymère en lui associant une micromolécule tensioactive. On peut ainsi conférer au polymère un caractère antistatique ou antibuée. Dans ce dernier cas les micromolécules tensioactives sont, par exemple, un monostéarate de pentaérythritol comme dans la demande de brevet japonais 72.52156, un monopalmitate de sorbitan comme dans la demande de brevet japonais 57.123239 ou encore un tensioactif fluorée ou siliconé comme dans les demandes japonaises 57 192445 et 58 76440. L'introduction de ces micromolécules tensioactives dans les polymères présente l'inconvénient de conduire à des compositions instables à l'humidité. Ces tensioactifs hydrosolubles sont extraits par l'eau de condensation, de sorte que leur efficacité est très limitée dans le temps.

L'oligomère selon l'invention, en tant que tensioactif, permet d'éviter cet inconvénient. Il est caractérisé par la formule

$$\begin{array}{l} \quad -(CR_1 - CH_2)_a- \\ \qquad | \\ O = C -(O - C_2H_4 - NH - \underset{\underset{O}{\|}}{C})_b-(O - C_2H_4)_n- \underset{\underset{(C_2H_4-O)_{n'}-H}{|}}{N} - R_2 -(Z)_m- R_3 \end{array}$$

dans laquelle
$R_1 = H$ ou $CH_3$
$R_2 = C_tH_{2t}$ avec $O \leqq t \leqq 18$

$$Z \text{ est choisi parmi } C_2H_4,\ C_2H_2 \text{ et } \begin{array}{l} CH - CH_2 \\ | \\ S - C_2H_4 - C_dF_{2d+1} \end{array}$$

avec $2 \leqq d \leqq 16$
m est un nombre entier tel que $1 \leqq m \leqq 3$
$R_3 = C_{t'}H_{2t'+1}$ avec $t' \geqq 1$ et $8 \leqq t + t' + 2m \leqq 22$
n et n' sont des nombres entiers, identiques ou différents,
tels que $2 \leqq n + n' \leqq 20$
b est un nombre entier égal à O ou 1
a est un nombre entier variant entre 2 et 500 sachant que préférentiellement les oligomères possèdent une majorité de chaines dans lesquelles $5 \leqq a \leqq 100$.

Cet oligomère associé en quantité de 0,1 à 5 et mieux de 0,2 à 0,5 % en poids par rapport au polymère permet d'en modifier les propriétés superficielles, en particulier il lui confère la propriété antibuée. Cette propriété est caractérisée en une modification du processus de condensation de l'eau sur la surface du polymère telle que la couche d'eau condensée ne diffuse plus, ou tout au moins atténue très sensiblement la diffusion de la lumière. En outre, cet oligomère quasiment inextractible par l'eau, confère une excellente stabilité dans le temps aux propriétés recherchées dans le polymère.

Comme déjà précisé l'oligomère tensioactif est obtenu par polymérisation d'un monomère résultant de la condensation ou de l'addition d'une amine grasse polyéthoxylée , ou d'un mélange d'amines grasses polyéthoxylées, sur un monomère à insaturation acrylique.

L'amine grasse polyéthoxylée est un produit dérivé de la chimie des acides gras. Sa fabrication est connue en elle-même. Elle résulte de l'addition d'oxyde d'éthylène sur une amine comme indiqué dans Organic Chemistry ; CRAM et HAMMOND ed. Mc Graw Hill 1959 p. 214. Cette amine peut par exemple être fabriquée selon la technique décrite dans le brevet USSR 598.876.

Ces amines grasses polyéthoxylées utilisées pour la fabrication de l'oligomère possèdent la formule générale

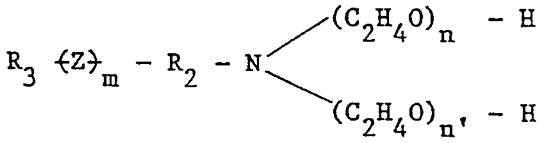

dans laquelle $R_2$, $R_3$, Z, n, n' et m sont tels que définis précédemment.

Industriellement ces amines grasses polyéthoxylées sont en fait constituées d'un mélange dont les chaines $R_3 -(Z)_m- R_2$ - possèdent une distribution centrée autour d'une valeur principale dépendant du corps gras initial à la préparation de l'amine. Ainsi la distribution caractérisant les amines dérivées de l'huile de coco est centrée sur une chaine alkyle de 12 atomes de carbone tandis que celle caractérisant les amines dérivées du suif est centrée sur une chaine alkylène de 18 atomes de carbone.

Si la chaine $R_3 -(Z)_m- R_2$ - possède au moins une insaturation éthylénique, il est possible de la greffer par un segment fluoré. Le mode préféré de greffage consiste à additionner radicalairement un thiol fluoré de formule générale $C_dF_{2d+1}$ - $C_2H_4$ - SH dans laquelle d est tel que défini précédemment. Ce thiol fluoré est un produit dérivé de la télomérisation du tétrafluoroéthylène dont la synthèse a été par exemple décrite dans la demande de brevet allemand 2 013 103. L'addition radicalaire du thiol fluoré consiste à amorcer la scission radicalaire du thiol par un agent de polymérisation ; les radicaux libres formés s'additionnent sur l'insaturation éthylénique de l'amine grasse avant ou après l'éthoxylation. Cette addition s'effectue en masse ou en milieu solvant non réactif, sous agitation et atmosphère inerte. La température de réaction, dépendant de la cinétique de décomposition de l'agent de polymérisation, peut varier de 60 à 140° C.

Le précurseur de l'oligomère tensioactif est un monomère polymérisable radicalairement de formule

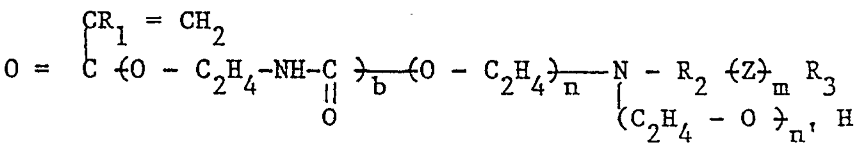

dans laquelle $R_1$, $R_2$, $R_3$, Z, b, m, n et n' sont tels que définis précédemment.

Ce monomère est obtenu :

- soit par condensation d'une amine grasse polyéthoxylée, ou d'un mélange d'amines grasses polyéthoxylées, avec l'acide acrylique ou méthacrylique, ou encore l'un de leurs dérivés tel le chlorure d'acryloyle, l'acrylate ou méthacrylate de méthyle,
- soit par addition d'une amine grasse polyéthoxylée, ou d'un mélange d'amines grasses polyéthoxylées, sur l'acrylate ou le méthacrylate du 2-isocyanatoéthyle de

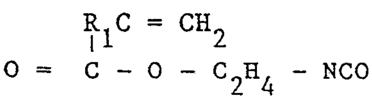

La condensation s'effectue, de préference en milieu solvant, sous agitation et atmosphère inerte à une température suffisante pour permettre le départ du produit de condensation. Il est possible d'ajouter au milieu réactionnel un catalyseur de condensation tel que l'acide paratoluènesulfonique ou le tétrabutylate de zirconium dans le cas d'une condensation avec l'acide acrylique ou méthacrylique.

Il est également possible d'ajouter au milieu réactionnel un inhibiteur de polymérisation tel que la méthylhydroquinone pour empêcher toute polymérisation des fonctions acrylates ou méthacrylates au cours de la condensation. L'évolution de cette condensation est mesurée par le dosage des fonctions hydroxyles de l'amine grasse polyéthoxylée.

La réaction est arrêtée lorsque la quantité de fonctions hydroxyles dosée est égale à la moitié de la quantité initiale de fonctions hydroxyles. On peut considérer que, statistiquement dans le monomère, une molécule d'amine grasse polyéthoxylée possède une fonction acrylate ou méthacrylate.

L'addition s'effectue de préférence en milieu solvant, sous agitation et atmosphère inerte à une température comprise entre 30 et 90° C et mieux entre 40 et 60° C. Pour accroitre la vitesse de réaction, on peut ajouter au milieu réactionnel un catalyseur utilisé habituellement dans la préparation de polyuréthanes tel que le dilaurate dibutyle d'étain. Il est également possible d'ajouter au milieu réactionnel un inhibiteur de polymérisation comme dans le cas de la condensation. La réaction d'addition est contrôlée par dosage des fonctions hydroxyles et est arrêtée lorsque la quantité dosée correspond à la moitié de la quantité initiale de fonctions hydroxyles. On peut considérer alors que, statistiquement dans le monomère, une molécule d'amine grasse polyéthoxylée possède une fonction acrylate ou méthacrylate.

L'oligomère selon l'invention est obtenu par polymérisation radicalaire du monomère précédent. La polymérisation radicalaire des monomères est connue. Toutefois, préférentiellement, la polymérisation radicalaire s'effectue en milieu solvant, sous agitation et atmosphère inerte en présence d'un agent de polymérisation tel que l'azobisisobutyronitrile ou le peroxyde de ditertiobutyle. La température de réaction,

dépendant de la cinétique de la décomposition de l'agent de polymérisation, peut varier de 40 à 130° C.

Dans la mesure où l'amine grasse polyéthoxylée ayant servi à la préparation du monomère peut être un mélange d'amines grasses à divers degrés de polyoxyéthylation, il n'est pas exclu que l'oligomère final soit un mélange d'oligomères répondant tous cependant à la formule générale précédente.

L'oligomère tensioactif est incorporé par tous moyens aux polymères thermoplastiques dont les propriétés superficielles doivent être modifiées. Habituellement cette incorporation s'effectue par malaxage de l'oligomère et du polymère de préférence à l'état fondu.

L'oligomère peut être associé à tous les polymères thermoplastiques, étant entendu que par polymères thermoplastiques il s'agit non seulement des homopolymères, mais encore des copolymères ou des mélanges de polymères et/ou de copolymères thermoplastiques. L'oligomère peut entre autre être associé au polyéthylène, au polychlorure de vinyle, ou encore au copolymère éthylène-acétate de vinyle.

Le polymère additionné d'oligomère peut ensuite être transformé selon toutes les techniques habituelles aux polymères thermoplastiques. La modification des propriétés superficielles du polymère thermoplastique est, dans les exemples suivants, évaluée par l'amélioration du comportement antibuée induite par l'oligomère tensioactif.

Cette évaluation s'effectue par différentes techniques.

Essai à chaud :

Un film de polymère thermoplastique est tendu horizontalement à 5 cm au-dessus d'un récipient contenant de l'eau en ébullition. On observe chronologiquement les différentes phases : apparition de gouttelettes de buée diffusantes, apparition de gouttes plus grosses qui tombent et se reforment, apparition d'un film d'eau continu. Le comportement antibuée le plus efficace est celui qui favorise l'apparition rapide d'un film d'eau continu qui ne diffuse plus la lumière. Cette propriété est en particulier recherchée dans le cas de films pour serres car une diffusion de la lumière diminue la fraction de rayonnement reçue par les plantes, ce qui est préjudiciable à leur croissance.

Essai à froid :

Des récipients contenant de l'eau, fermés par un film tendu de polymère thermoplastique sont placés dans un congélateur à -18° C. On observe chronologiquement les différentes phases au cours du refroidissement. Ces récipients sont laissés 20 h. à -18° C puis retirés du congélateur. On observe chronologiquement les différentes phases de réchauffement. Dans cet essai à froid, un comportement antibuée efficace se traduit par la formation d'un film d'eau continu.

Mesure des tensions d'adhésion :

Ces mesures sont effectuées sur une surface moyenne de 2 $cm^2$ de film de polymère thermoplastique par trempage dans l'eau. On détermine la tension d'adhésion à l'avancée ($T_A$) du film dans l'eau et la tension d'adhésion au retrait ($T_R$) du film. La différence entre ces deux tension $\Delta W_{SL} = T_A - T_R$ représente l'hystérésis du mouillage. Ces valeurs sont exprimées en millinewton par mètre (mN/m).

On mesure également la tension superficielle de l'eau après ce trempage. Si cette tension superficielle ($\gamma_L$)est plus faible que celle de l'eau pure : 72 mN/m, cela signifie qu'une partie de l'agent antibuée a migré dans l'eau au cours du trempage : cette migration est préjudiciable à l'efficacité à long terme de cet agent antibuée. La faible hydrosolubilité de l'oligomère tensioactif se traduit par une variation quasi négligeable de la tension superficielle de l'eau après trempage.

Cinétique de condensation :

Un récipient d'eau est fermé par un film tendu de polymère thermoplastique et est maintenu à 33° C. Le phénomène de condensation de la vapeur d'eau est suivi par microscopie optique (grossissement 250). On observe la cinétique de coalescence des gouttelettes pour former un film continu d'eau.

De l'ensemble des évaluations, il ressort que l'oligomère tensioactif induit un comportement antibuée au moins comparable aux agents antibuée du commerce tel que l'ATMER®. L'intérêt de cet oligomère tensioactif, contrairement à ces agents antibuée commerciaux, se trouve dans la quasi absence d'extraction par l'eau de condensation. Cette extraction permanente pour les produits commerciaux limite leur efficacité dans le temps, contrairement à celle de l'oligomère.

Les exemples suivants illustrent l'invention sans la limiter.

EXEMPLE 1

Dans un réacteur de 2 litres à trois tubulures on introduit 300 g. de NORAMOX 05® qui est une amine grasse polyéthoxylée dont la chaine grasse $R_3 \left( Z \right)_m R_2$ est principalement un motif oleyle avec $R_3$ le groupement $CH_3 \left( CH_2 \right)_7$, Z le groupement CH = CH, $R_2$ le groupement $(CH_2)_8$, et le nombre de motifs éthoxylés (n + n') égal à 5. L'indice d'hydroxyle de ce produit est de 262,5. Au NORAMOX 05® on ajoute dans le réacteur 90,94 g. d'acide acrylique, 2,7 g. d'acide paratoluènesulfonique, 0,6 g. d'hydroquinone et 1200 $cm^3$ de toluène. L'ensemble est porté au reflux, sous agitation et balayage d'azote. L'indice d'acide est périodiquement déterminé. La réaction est arrêtée quand l'indice d'acide est proche de la valeur théorique correspondant à la monoestérification de l'amine grasse polyéthoxylée. Le solvant est éliminé par distillation sous léger vide et l'acide acrylique résiduel éliminé à 150° C sous vide de 110 Pa. L'indice d'hydroxyle du produit obtenu est de

144,8.

Dans un réacteur de 2 litres à trois tubulures on introduit 328 g. du produit obtenu, 6 g. d'azobisisobutyronitrile et 600 $cm^3$ d'alcool éthylique. L'ensemble est maintenu 6 heures à 72° C sous agitation et atmosphère inerte. L'éthanol est ensuite distillé d'abord sous léger vide à 80° C puis pendant 10 minutes à 150° C sous un vide de 66 Pa.

On récupère le produit selon l'invention dont la composition est évaluée par analyse chromatographique par perméation de gel en utilisant un étalonnage au polystyrène. Le produit est constitué de chaines acryliques dont les masses sont représentées par des valeurs de "a" comprises entre 4 et 25, centrées majoritairement sur 4 et 7.

EXAMPLE 2

Dans un réacteur d'un litre on introduit 200 g. de NORAMOX 05® 468,5 g. de thiol fluoré de formule $C_6F_{13}$ - $C_2H_4$ - SH et 4,84 g. d'azobisisobutyronitrile. L'ensemble est maintenu 7 h. à 77° C sous agitation et atmosphère d'azote. Le thiol fluoré excédentaire est ensuite distillé à 130° C sous léger vide. Le rendement de greffage du thiol fluoré sur l'insaturation de l'amine grasse polyéthoxylée est de 92,8 %. L'indice d'hydroxyle de l'amine obtenu est de 150.

Dans le réacteur de 2 litres à trois tubulures on introduit 300 g. de l'amine greffée précédente, 46,3 g. d'acide acrylique, 1,53 g. d'acide paratoluènesulfonique, 0,6 g. d'hydroquinone et 1200 $cm^3$ de toluène. L'ensemble est porté au reflux, sous balayage d'azote et agitation. L'indice d'acide est périodiquement déterminé. La réaction est arrêtée après 19 heures de reflux. Le toluène et l'acide acrylique résiduel sont extraits par distillation sous vide de 110 Pa. L'indice d'hydroxyle du produit obtenu est de 79,8.

Dans un réacteur de 2 litres à trois tubulures, on introduit 302 g. du monomère obtenu, 6 g. d'azobisisobutyronitrile et 600 $cm^3$ d'éthanol. Le mélange est maintenu 6 h. à 70° C, sous agitation et balayage d'azote. On introduit ensuite 2 g. d'azobisisobutyronitrile et on poursuit la polymérisation pendant 4 h. à 70° C. L'éthanol est ensuite éliminé par distillation.

Le produit est analysé dans les conditions de l'Exemple 1. Il est constitué de chaines acryliques dont les masses correspondent à des valeurs de "a" comprises entre 4 et 63 centrées majoritairement sur 4 et 10.

EXEMPLE 3

Au moyen d'une extrudeuse (type BUSS PR46) on mélange et granule du copolymère éthylène-acétate de vinyle (1020 VN3 de la Société ATOCHEM) avec 5000 ppm d'oligomère de l'Exemple 1. La température de malaxage est de 175 à 190° C pour un débit de 20 kg/h. 20 kg de granulés ainsi préparés sont extrudés sous forme de gaine d'une largeur de 500 mm au moyen d'une extrudeuse TROESTER équipée d'un embout malaxeur et d'une filière GLOENCO de 100 mm de diamètre. Les températures d'extrusion s'échelonnent de 140 à 160° C. La vitesse de rotation de la vis d'extrusion et la vitesse de tirage de la gaine sont ajustées de façon à ce que la gaine soit constituée d'un film de 100 microns d'épaisseur (Echantillon 2).

Un essai témoin est réalisé dans les mêmes conditions sans ajout d'oligomère (Echantillon 1).

Un essai est réalisé dans les mêmes conditions que précédemment en granulant le polyéthylène avec 5000 ppm de l'oligomère de l'exemple 2 (Echantillon 3).

Evaluation antibuée : Essais à chaud et à froid :

Des films découpés dans les échantillons 1, 2 et 3 sont tendus, horizontalement à 5 cm au-dessus d'un récipient contenant de l'eau en ébullition.

Pour l'Echantillon 1, on observe l'apparition instantanée de buée, puis de gouttelettes après 32 secondes d'exposition, puis de gouttes à 5 minutes 50 secondes. Après 25 minutes, les grosses gouttes tombent du film et le processus de condensation recommence.

Pour l'Echantillon 2, on observe l'apparition de gouttelettes après 30 secondes d'exposition, l'apparition de gouttes après 1 minute 25 secondes. Après 2 minutes 50 secondes, les gouttes se rassemblent pour former un film d'eau continu qui ne diffuse pas la lumière.

Pour l'Echantillon 3, on observe la formation quasi instantanée d'un film d'eau continu.

Des films des Echantillons 1, 2 et 3 obturent des récipients qui sont placés dans un congélateur à -18° C.

Pour l'Echantillon 1, il se forme instantanément de la buée, puis des gouttelettes après 7 minutes et finalement des gouttes après 18 minutes de refroidissement.

Pour l'Echantillon 2, on constate l'apparition des mêmes phénomènes, mais les gouttelettes n'apparaissent qu'après 6 minutes et les gouttes après 15 minutes.

Pour l'Echantillon 3, on observe la formation d'une légère couche de buée qui laisse place à un film d'eau continu après 2 minutes de refroidissement.

Les récipients sont laissés 20 h. à -18° C puis remis à la température ambiante.

Pour l'Echantillon 1, on constate, après 10 minutes, l'apparition de gouttes sur la face interne du film et la présence de buée sur la face externe.

Pour l'Echantillon 2, on constate, après 10 minutes, l'apparition de gouttes sur la face interne du film et de gouttelettes sur la face externe ; globalement le film diffuse moins la lumière que l'Echantillon 1.

Pour l'Echantillon 3, le film reste constamment transparent.

EXEMPLE 4

Dans les conditions de l'Exemple 3 on prépare des granulés de copolymère éthyléne-acétate de vinyle (1005 VL4 de la Société ATOCHEM) contenant 5000 ppm de l'oligomère de l'Exemple 1.

20 kg de granulés préparés sont extrudés sous forme de gaine d'une largeur de 350 mm. au moyen d'une extrudeuse KAUFMAN PKH 28-65 équipée d'un embout malaxeur. La température de la matière est de 210°C. La vitesse de rotation de la vis d'extrusion et le taux de gonflage de la gaine sont réglés de manière à ce que l'épaisseur de la gaine soit de 150 microns (Echantillon 5).

Un essai témoin est réalisé dans les mêmes conditions sans ajout d'oligomère (Echantillon 4).

Un essai est réalisé dans les mêmes conditions que précédemment en granulant le copolymère avec 5000 ppm de l'oligomère de l'Exemple 2 (Echantillon 6).

Un essai est réalisé dans les mêmes conditions que précédemment en granulant le copolymère avec 5000 ppm d'ATMER 184®, produit commercialisé comme agent antibuée (Echantillon 7).

Un essai comparatif est réalisé dans les mêmes conditions que précédemment en granulant le copolymère avec 5000 ppm de NORAMOX 05® (Echantillon 8).

Evaluation antibuée :

Des films découpés dans les Echantillons 4, 5, 6 et 8 sont tendus horizontalement à 5 cm. au-dessus d'un récipient contenant de l'eau en ébullition.

Pour l'Echantillon 4, il se forme instantanément de la buée, puis des gouttelettes après 50 secondes d'exposition et enfin des gouttes après 5 minutes 40 secondes. Les gouttes tombent après 10 minutes et le cycle de condensation recommence.

Pour l'Echantillon 5, on observe l'apparition de gouttelettes après 15 secondes d'exposition, puis des gouttes après 2 minutes 50 secondes qui s'étalent pour former un film d'eau continu.

On observe les mêmes phénomènes pour les Echantillons 6 et 8 sauf que l'apparition des gouttes se fait respectivement après 2 minutes 30 secondes et 4 minutes.

Cinétique de condensation :

La cinétique de condensation est mesurée sous microscope optique.

Des films découpés dans les Echantillons 4, 5, 6, 7 et 8 obturent des récipients d'eau maintenus à 33°C. La forme sous laquelle l'eau se condense est observée après 15 secondes, 5, 15 et 60 minutes d'exposition. Les observations sont regroupées dans le tableau suivant ; le terme "flaque" désigne une goutte d'eau étalée, peu diffusante, dont le contour est irrégulier contrairement à celui des gouttes qui est circulaire. Les formes de condensation sont identiques sur les deux faces du film sauf pour l'Echantillon 8 qui présente une dissymétrie.

| | Formes de condensation observées après | | | |
|---|---|---|---|---|
| | 15 secondes | 5 minutes | 15 minutes | 60 minutes |
| ECHANTILLON 4 | gouttelettes de 2 microns | gouttelettes de 8 microns | gouttes de 15 à 20 microns | gouttes de 60 à 70 microns |
| ECHANTILLON 5 | gouttelettes de 1 à 2 microns | "flaques" de 20 microns | "flaques" d'environ 60 à 80 microns | "flaques" jointives non diffusantes |
| ECHANTILLON 6 | gouttelettes de moins de 1 micron | "flaques" de 40 à 60 microns | "flaques" jointives d'environ 120 microns non diffusantes | "flaques" jointives non diffusantes |
| ECHANTILLON 7 | gouttelettes de moins de 1 micron | "flaques" de 40 à 60 microns | "flaques" jointives d'environ 120 microns non diffusantes | "flaques" jointives non diffusantes |
| ECHANTILLON 8 1ère face | gouttelettes de 1 à 2 microns | gouttelettes de 4 à 8 microns | "flaques" de 12 à 16 microns | "flaques" de 40 à 60 microns |
| ECHANTILLON 8 2ème face | gouttelettes de 1 à 2 microns | gouttelettes de 4 à 8 microns | gouttelettes de 8 à 12 microns | gouttelettes de 16 à 20 microns |

Seuls les Echantillons 5, 6 et 7 ont un comportement antibuée intéressant.

Evaluation de la tenue à l'eau :

L'efficacité à long terme d'un additif antibuée est liée au fait qu'il est peu ou pas entrainé et dissous par l'eau de condensation.

On peut évaluer cette efficacité par la méthode suivante :

on mesure la tension d'adhésion T d'un film dopé par un agent antibuée dans de l'eau bidistillée puis on mesure la tension superficeille $\gamma_L$ de cette eau par la méthode de l'étrier : plus l'additif est extractible par l'eau, plus la tension superficielle de l'eau sera inférieure à sa valeur initiale (72 mN/m).

La mesure de la tension d'adhésion est effectuée selon le protocole expérimental décrit par T et L.

GUASTALLA. (Journal de Chimie Physique 49 n° 5 1951 et 51 n° 10 1954) et réalisée avec un tensiomètre automatique LAUDA.

La méthode de l'étrier est réalisée selon la norme DIN 53194.

Les résultats obtenus sur les échantillons 4 à 7 sont regroupés dans le tableau suivant : $T_A$ et $T_R$ sont respectivement les tensions d'adhésion à l'avancée et au retrait et $\gamma_L$ la tension superficielle de l'eau après ce cycle de mouillage.

| | $T_A$ mN/m * | $T_R$ mN/m * | $\gamma_L$ mN/m * |
|---|---|---|---|
| ECHAN-TILLON 4 | - 22 | 8 | 72 |
| ECHAN-TILLON 5 | - 16 | 6 | 71 |
| ECHAN-TILLON 6 | - 14 | 11 | 72 |
| ECHAN-TILLON 7 | 8 | 22 | 60 |

* mN/m = milliNewton par mètre

On constate que la tension superficielle de l'eau bidistillée n'évolue pas ou peu ($\gamma_{L_0}$ = 72 mN/m) pour les Echantillons 4, 5 et 6 tandis qu'elle chute à 60 mN/m pour l'Exhantillon dopé par de l'ATMER 184.

## Revendications

1. Oligomère de formule

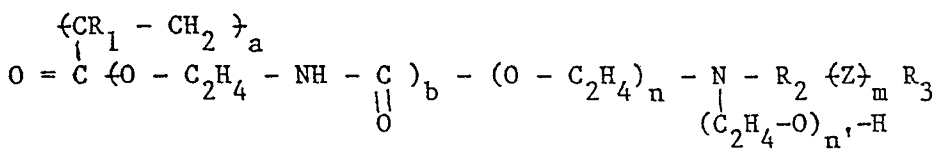

dans laquelle
$R_1$ = H ou $CH_3$
$R_2$ = $C_tH_{2t}$ avec $0 \leqq t \leqq 18$

Z est choisi parmi $C_2H_4$, $C_2H_2$ et 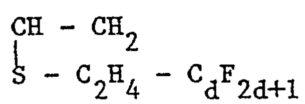

avec $2 \leqq d \leqq 16$
m est un nombre entier tel que $1 \leqq m \leqq 3$
$R_3$ = $C_{t'}H_{2t'+1}$ avec $t' \geqq 1$ et $8 \leqq t + t' + 2m \leqq 22$
n et n' sont des nombres entiers, identiques ou différents,
tels que $2 \leqq n + n' \leqq 20$
b est un nombre entier égal à O ou 1
a est un nombre entier variant entre 2 et 500

2.- Oligomère selon la revendication 1 caractérisé en ce que n et n' sont au moins égaux à 1.

3.- Oligomère selon l'une des revendications 1 ou 2 caractérisé en ce qu'il possède une majorité de chaines dans lesquelle $5 \leqq a \leqq 100$.

4.- Mélange d'oligomères des revendications 1 à 3.

5.- Procédé de fabrication de l'oligomère selon l'une des revendications 1 à 4 consistant en une polymérisation d'un monomère résultant de la condensation ou de l'addition d'une amine grasse polyéthoxylée ou d'un mélange d'amines grasses polyéthoxylées sur un monomère insaturé caractérisé en ce que le monomère répond à la formule

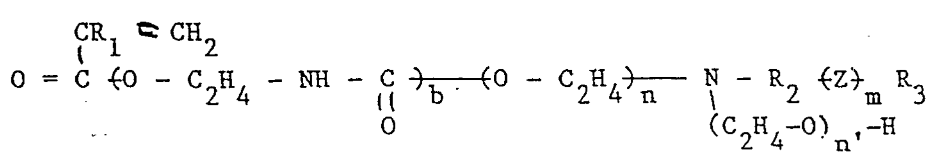

dans laquelle $R_1$, $R_2$, $R_3$, Z, b, m, n et n' sont tels que définis dans l'une des revendications 1 ou 2.

6.- Procédé selon la revendication 5 caractérisé en ce que le monomère résulte de la condensation d'une amine grasse polyéthoxylée, ou d'un mélange d'amines grasses polyéthoxylées, avec l'acide acrylique ou méthacrylique ou encore un de leurs dérivés.

7.- Procédé selon la revendication 6 caractérisé en ce que le monomère résulte de l'addition d'une amine grasse polyéthoxylée, ou d'un mélange d'amines grasses polyéthoxylées, sur l'acrylate ou le méthacrylate du 2-isocyanatoéthyle.

8.- Procédé selon l'une des revendications 5 à 7 caractérisé en ce que le monomère contient statistiquement une fonction acrylate ou méthacrylate par molécule d'amine grasse polyéthoxylée.

9.- Procédé selon l'une des revendications 5 à 8 caractérisé en ce que le monomère résulte de la condensation ou de l'addition d'un mélange d'amines grasses à différents degrés de polyoxyéthylation sur un monomère à insaturation acrylique ou méthacrylique.

10.- Tensioactif selon l'une des revendications 1 à 9.

11.- Application comme antibuée dans les films de polymère thermoplastique de l'oligomère des revendications 1 à 10.

12. Mélange à base de polymère thermoplastique caractérisé en ce qu'il contient de 0,1 à 5 % en poids par rapport au polymère d'oligomère d'une des revendications 1 à 11.